# EUROPEAN PATENT APPLICATION

(11) **EP 4 693 327 A1**
(43) Date of publication of application: **11.02.2026**
(21) Application number: 24193429.8
(22) Date of filing: 07.08.2024
(51) Int. Cl.: G16H 30/00

(54) **METHODS FOR PROCESSING IMAGE DATA**

(71) Applicant: SpatialX Diagnostics Ltd, London W11 4JA (GB)
(72) Inventor: Shaker, Noor, London, W11 4JA (GB); Shaker, Mohammad, London, W11 4JA (GB)
(74) Representative: Potter Clarkson

(57) **Abstract**

A method for processing medical image data for a display device, the method comprising: receiving medical image data, the medical image data comprising at least one image and initial annotation data corresponding to at least one image, the initial annotation data being defined by a first plurality of values; receiving a set of resource constraint parameters; receiving instructions to select a subregion of the image as a display subregion; generating display annotation data to represent the initial annotation data, the display annotation data corresponding to the display subregion and being defined by a second plurality of values, wherein the second plurality is generated from the first plurality and the number of values in the second plurality is determined by the resource constraint parameters; and providing instructions to display the display subregion overlaid with the display annotation data on the display device.

## Description

### FIELD OF THE INVENTION

The invention relates to systems and methods for optimising processing of annotated medical image data for display on a display device.

### BACKGROUND TO THE INVENTION

Images are increasingly processed using automated analysis techniques. This is particularly true in the context of medical imaging, where the utilization of deep learning techniques is becoming increasingly prevalent for tasks such as classification, segmentation, and clinical decision-making. Medical image data is often annotated to facilitate analysis. Annotation involves recognising and, in some way, classifying or highlighting features within the image. Annotation may be performed manually, i.e. by a human annotator, or automatically by an annotation system trained to recognise and annotate features.

Deep learning methods for computer vision have revolutionized the field of medical imaging by offering powerful tools for analysing complex datasets and extracting meaningful insights. However, the effective utilization of these techniques is contingent upon the availability of high-quality annotated data. Traditional approaches to image annotation, preprocessing and data preparation often work on relatively small images. Medical images pose challenges due to the size and dimensionality which can be a number of magnitudes larger than images in other domains. For example, images in the ImageNet dataset, a flagship dataset for training deep learning, are of size 460 x 390 pixels, while an average whole-slide histopathology image may contain 100,000 x 100,000 pixels. In addition to the images themselves, the number and complexity of annotations associated with image data can be very large. Each image in the ImageNet database, of the kind discussed above, may have tens of thousands of associated annotations and gigabytes' worth of associated annotation information.

It is often desirable for users to visualise the images and associated annotations on a display device, in order to analyse the medical image data. In some cases, a user may wish not just to visualise the annotations but to edit or add to them. However, the large size of medical images and their associated annotation data imposes a limitation when visualising the information on a display device. The computational resources required to process and render the information means that only computers with considerable computational resources are suitable.

Accordingly, there is a need for systems and methods which allow annotated medical image data to be visualised and edited more easily.

### SUMMARY OF THE INVENTION

According to a first aspect, there comprises a method for processing medical image data for a display device, the method comprising: receiving medical image data, the medical image data comprising at least one image and initial annotation data corresponding to the at least one image, the initial annotation data being defined by a first plurality of values; receiving a set of resource constraint parameters, the resource constraint parameters representing computational resources of the display device; receiving instructions to select a subregion of the image as a display subregion; generating display annotation data to represent the initial annotation data, the display annotation data corresponding to the display subregion and being defined by a second plurality of values, wherein the second plurality of values is generated from the first plurality of values and the number of values in the second plurality of values is determined by the resource constraint parameters; and providing instructions to display the display subregion overlaid with the display annotation data on the display device.

In conventional approaches, the entirety of the medical image data, including the whole image and all annotations, is loaded into memory and processed for display on the display device. However, this is extremely computationally intensive due to the size of the image and number and complexity of the annotation data. The displayed image and data load slowly and respond sluggishly to user inputs, and the display device is susceptible to crashing due to the high computational workload.

By contrast, the method of the invention advantageously optimises the display of the medical data for the display device, by adapting the level of complexity of the displayed annotations according to the resources made available by the display device. If the available resources of the display device are high, then the number of values in the second plurality of values is high, and hence the number and complexity of the displayed annotations can be higher. Indeed, in some examples the computational resources may be sufficient that the entire first plurality of values are retained for the second plurality of values - i.e. all annotations in the initial annotation data may be loaded for display on the display device. In other examples, the computational resources are more limited. In such examples, the number of values in the second plurality is smaller than the number of values in the first plurality. Rather than processing the entirety of the first plurality in order to display all the initial annotation data, the smaller and lower-complexity second plurality is processed to provide a more computationally and potentially also visually efficient representation of the annotation data.

By optimising the utilisation of computational resources, the method enables annotated medical image data to be visualised on resource-limited devices such as notebook computers, smartphones, or tablet computers. In addition, the optimised resource utilisation allows the method to run faster on a particular device, thereby reducing latency. Low latency is particularly useful when the display device is a touchscreen device, as users are more sensitive to slow responsiveness when using their fingers to directly manipulate a display. Rather than having to wait for access to a powerful desktop computer, a user can visualise and potentially also manipulate annotated medical image data using their personal touchscreen device. Thus, the method enables medical image data to be performed on a wider range of devices, and with greater intuitiveness and ease of use.

The first and second plurality of values respectively define the initial annotation data and display annotation data. Annotation data may comprise a plurality of annotations, with each annotation being defined by values representing properties of the annotation, such as the position of the annotation, the size and geometry of the annotation, and/or the class of the annotation. Values in the first and second plurality can also represent aggregate properties of the annotation data, rather than specific individual annotations.

Furthermore, reducing the complexity of the initial annotation data may be advantageous for visualising the image and annotation data. The initial annotation data may be sufficiently complex and dense that it would be difficult for a user to understand if it were all overlaid on the image at once. This is particularly true when viewing an image at a comparatively low magnification, as the number of annotations within the field of view may be so large that it is no longer possible to see the underlying image. By contrast, the second plurality can be generated to ensure that the display annotation data overlaid over the display subregion is not overly complex. This enables users to analyse the image and annotation data with greater ease, simplifying and accelerating their workflow.

The image may comprise a Whole Slide Image (WSI). Such images are high-resolution photographs or scans of tissue, and are often on the order of gigabytes or even larger in size.

In some examples, there may comprise intermediate mapping steps when generating the second plurality from the first plurality. For example, the values of the first plurality may be mapped to generate an intermediate plurality of values, and then the intermediate plurality of values is mapped to generate the second plurality of values.

Optionally, the second plurality comprises a subset of the first plurality. That is, the second plurality is generated by selecting values from the first plurality. For example, the method may select values representing only a certain proportion (e.g. 10%) of all the annotations in the initial annotation data. The size of the subset may be determined by the resource constraint parameters. Selecting values to form a subset is advantageously a simple and computationally efficient way of reducing the size and complexity of the initial annotation data.

Optionally, the first plurality comprises overlay size values defining overlay sizes for annotations in the initial annotation data, and generating the second plurality comprises comparing the overlay size values to a threshold, the threshold being determined with reference to the resource constraint parameters. The threshold may alternatively or additionally be determined based on the selection of the display subregion.

The overlay size values may represent the area or radius of the corresponding annotation on the display subregion. By comparing the overlay size values to a threshold, the method can determine whether the annotation should be used to generate the second plurality.

For example, generating the second plurality may comprise selecting, for the second plurality, values from the first plurality representing annotations with an overlay size value within the threshold range. This may ensure that computational resources are not wasted. One example of such a scenario is the processing of the first plurality corresponding to annotations which are too large or small to be usefully displayed over the selected display subregion. The threshold may be determined based on user-configurable parameters such as zoom and detail level, as well as the resource constraint parameters. For example, as a user provides instructions to zoom in on a section of the image, the threshold range may decrease so that previously excluded smaller annotations are included, and previously included larger annotations are excluded.

Optionally, generating the second plurality comprises generating values defining a spatial representation, the spatial representation representing values from the first plurality representing annotations with an overlay size value lower than the threshold.

Rather than showing each individual annotation, the spatial representation provides a visual representation of the spatial density and distribution of annotations in aggregate. One example of such a spatial representation is a colourmap or a heatmap. A colormap uses multiple colours or shades to indicate different levels of annotation across the display subregion. Other spatial representations are also envisioned, such as bubble maps or even three-dimensional representations. The spatial representation can be defined by a smaller number of values than all of the individual annotations it represents, thereby reducing computational workload. Furthermore, the spatial representation allows the distribution of small-size annotations to be visualised, without having to zoom in on the display subregion to a level where the small-size annotations are individually discernible and without having to load them in a zoom-out view.

The spatial representation may also be generated by one or more intermediate mappings or selections from the first plurality.

Optionally, the first plurality comprises geometry values defining geometries for annotations in the initial annotation data, and generating the lower-complexity annotation data comprises generating a lower-resolution set of geometry values for the annotations.

Reducing the resolution of annotation geometry may significantly reduce computational workload, while still allowing the location and approximate geometry of the annotation to be visualised. In some examples, the geometry values define the geometry of the annotation by defining the location of the vertices making up a polygon. To reduce the resolution, the number of vertices may be constrained by selecting a subset of the vertices for each annotation geometry, or by generating values representing a new, smaller set of vertices for that annotation or by representing the annotation using a different form of geometry such as replacing a polygon with a circle or another simpler shape. Annotation geometries may be replaced by a shape selected from a set of simplified shapes (e.g. a set including dots, squares, triangles and circles), with the simplified shape being selected based on which of the simplified shapes most closely resembles the original annotation geometry.

In some instances, alternate representations such as Bezier curves may be generated based on an initial polygon annotation geometry. A smaller number of values may be able to characterise a Bézier curve to parametrise a larger number of values defining a polygon annotation geometry.

Optionally, the method further comprises: identifying a first cluster plurality of values from the first plurality representing a cluster of annotations within the annotation data, and generating a second cluster plurality of values representing the cluster from the first cluster plurality.

A cluster of annotations may comprise a region of the display subregion with a greater-than-average spatial density of annotations. In some examples, clusters may only be identified if they comprise only or primarily annotations meeting certain conditions, such as being within a certain overlay size or of a particular class. Generating a second cluster plurality of values representing the cluster allows the cluster to be visualised in the display annotation data without needing to store the values representing each individual annotation in the cluster.

Optionally, the first plurality comprises class values representing annotation classes in the initial annotation data, and the second plurality is generated with reference to the class values. For example, each annotation class could represent a cell class, such as tumour cell vs. non-tumour cell, or the tissue type of the annotated tissue.

Optionally, the method further comprises: identifying, from the class values, a subset of classes of annotations within the initial annotation data; and selecting for the second plurality, using the class values, values from the first plurality representing annotations within the subset of classes. In other words, the second plurality may be selected such that it comprises a variety of annotations of different classes, or even a selection of all of the different annotation classes within the initial annotation data. This ensures that the second plurality can accurately represent and visualise the different annotation classes within the initial annotation data, even if the number of values in the second plurality is significantly smaller than in the first plurality.

Optionally, the method further comprises: identifying, using the class values, a first class region within the display subregion comprising annotations having a first specified annotation class; and selecting for the second plurality, using the class values, values from the first plurality representing a subset of the annotations in the first class region having the first specified annotation class.

The inventors have observed that different regions of a medical image are often predominantly or at least significantly comprised of annotations of particular classes, particularly if the medical image has been automatically annotated rather than manually annotated. For example, a region of a medical image comprising a tumour may comprise hundreds or even thousands of annotations classed as "tumour cell". Therefore, the display annotation data can be considerably simplified by identifying class regions, and then selecting or generating a smaller number of values for the second plurality representing annotations of that class in that class region.

Optionally, the method further comprises identifying, using the class values, an intersection subregion where the first class region intersects with a second class region, the second class region comprising annotations having a second specified annotation class, wherein selecting values for the second plurality comprises selecting values representing a subset of the annotations in the intersection region having the first or second specified annotation class.

By selecting values for the second plurality representing annotations in the intersection regions, the display annotation data will comprise annotations at or close to regions where one class of annotations changes to another, such as the boundaries of a tumour region. This enables the edges and geometry of different class regions to be clearly identified on the display subregion, even with a small number of displayed annotations. This is particularly advantageous when visualising medical images where interactions between modalities are important, as emphasis is given on viewing annotations that are at the intersection boundaries between different classes of annotations.

Techniques such as QuadTree may be implemented to determine the number of annotations of a particular class clustered within a given region, and/or the spatial location of different clusters of annotations.

Optionally, the method comprises determining an adjacent subregion of the image to the selected display subregion, wherein the display annotation data further corresponds to the display subregion and the adjacent subregion. That is, the second plurality is generated so as to comprise values representing annotations in the adjacent subregion as well as the display subregion. While the display subregion is displayed on the display device, the adjacent subregion is a region geographically proximal or even bordering the display subregion - i.e. a region with high likelihood that a user decides to pan or otherwise navigate to. By populating the annotation data with annotations for the adjacent subregion, the method thus pre-prepares annotations and image data in case the user navigates to the adjacent subregion, thereby reducing latency. However, computational workload is still reduced because the display device still may not need to load the entire image and corresponding annotations, only the display subregion and one or more adjacent subregions. In a similar manner, the method may automatically free (deallocate) annotations from the device resources when the user navigates away from a region. This keeps the resources allocated optimised.

The display device may comprise an interface allowing the user to modify the user-editable parameters. Modification may include altering the values of the second plurality so as to add, remove, or modify properties (such as size, class or geometry) of annotations on the display subregion.

Optionally, the method further comprises: receiving modification instructions from a user interface device; associating the modification instructions with a value of the second plurality; using the associated value of the second plurality, associating the modification with a value of the first plurality; and modifying the associated value of the first plurality in response to the modification instructions. In other words, the method can propagate a modification made to the display annotation data back to the initial annotation data, and make a corresponding modification. This allows a user to modify annotations in the initial annotation data, by interacting with annotations in the display annotation data via the display device and user interface device.

According to a second aspect, there comprises a data processing device configured to carry out the method of the first aspect.

According to a third aspect, there comprises a (non-transitory) computer-readable storage medium comprising instructions which, when executed by a data processing device, cause the data processing device to carry out the method of the first aspect.

According to a third aspect, there comprises a computer program comprising instructions which, when executed by a data processing device, cause the data processing device to carry out the method of the first aspect.

### BRIEF DESCRIPTION OF THE DRAWINGS

Figure 1 is a process flow chart illustrating an exemplary method.
Figure 2 illustrates steps of an exemplary method for generating a display subregion and display annotation data. Figure 2a illustrates a simplified example of medical image data and initial annotation data, Figure 2b shows the selection of a display subregion and Figure 2c shows the generation of display annotation data.
Figure 3 illustrates an exemplary display device displaying a display subregion, display annotation data and a user interface.
Figure 4 illustrates an example of the method in which geometrical annotations are simplified. Figures 4a and 4c show a display subregion and initial annotation data, while Figures 4b and 4d show a display subregion with display annotation data comprising simplified-geometry annotations.
Figure 5 illustrates an example of the method in which annotations are simplified based on overlay size. Figure 5a shows a display subregion and initial annotation data, while Figure 5b shows the display subregion with display annotation data. Figure 5c shows another example of a display subregion and display annotations comprising a spatial representation.
Figure 6 illustrates an example of the method in which annotations are simplified by identifying clusters, class regions, and/or intersection regions. Figures 6a and 6c show a display subregion and initial annotation data, Figure 5b shows the display subregion with display annotation data generated by identifying clusters, and Figure 6c shows the display subregion with display annotation data generated by identifying intersection regions.
Figure 7 illustrates the components of an exemplary system suitable for implementing an exemplary method.

### DETAILED DESCRIPTION

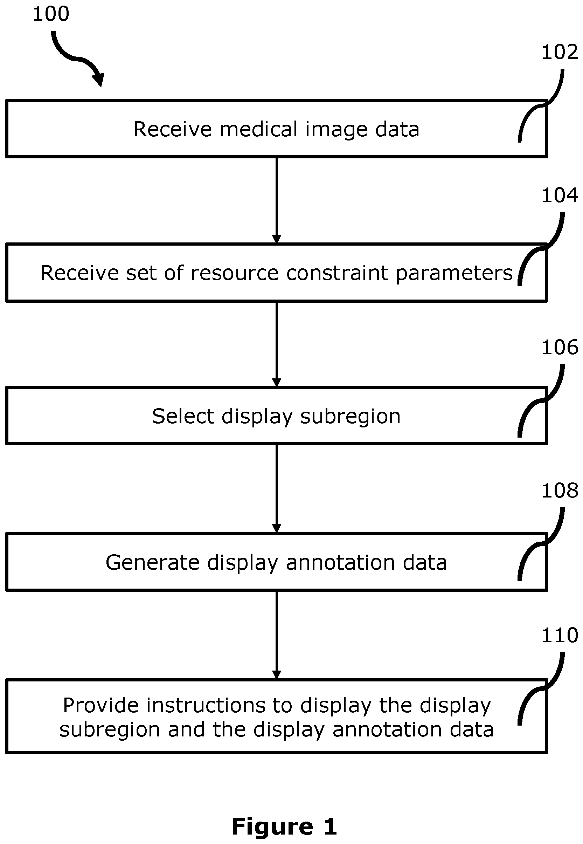

Figure 1 illustrates a method 100 for processing medical image data for a display device according to the present disclosure.

In step 102, medical image data is received, the medical image data comprising at least one image and initial annotation data corresponding to the at least one image, the initial annotation data being defined by a first plurality of values. The medical image data may be received from a remote digital storage location, or from memory storage attached to, or part of, the display device. Images may be provided in one format (such as ndpi, svs, tiff), and may be converted to another format (such as dzi) for further processing.

In step 104, a set of resource constraint parameters is received, the resource constraint parameters representing computational resources constraining the operation of the display device. The resource constraint parameters may also be received from a local server or a remote location.

In some examples, the resource constraint parameters may represent, or be associated with, the properties of the computational resources available to the display device. For example, the resource constraint parameters may represent available computer memory (RAM), processor power (CPU and/or GPU), display size, or display resolution. In some examples, the resource constraint parameters represent physical constraints, such as the total system memory (RAM) or CPU/GPU calculations-per-second allocation made available by the display device's hardware, or the remaining level of RAM or CPU/GPU allocation still available (i.e. not currently in use by another application).

In some examples, the method 100 may be implemented by a virtual machine or another application operating on a device. The resource constraint parameters may therefore represent the total computational resources or available proportion thereof made available to the virtual machine or application. The resource constraint parameters may also include a specification for a display element of the display device, which provides information about the level of computational resources required to drive the display element. Such a specification may include, for example, one or more of the current resolutions, refresh rate and max resolution of the display element. In some examples, the specification is set by the hardware of the display device, but in other examples a virtual specification may be defined (e.g. deliberately specifying a lower resolution or refresh rate in order to reduce computational workload).

In some examples, processing may take place on a remote server, such as the Cloud. For example, the method 100 may be carried out on the Cloud, with a user providing instructions and viewing the display subregion and annotations via an application such as a web browser on a mobile display device. In such cases, the resource constraint parameters may represent the computational resources made available for the display device by the remote server, or a constraint imposed by the communication medium between the mobile display device, such as the user's internet speed or maximum bandwidth or bitrate.

Alternatively or additionally, the resource constraint parameters may represent user-defined or user-editable constraints. That is, the complexity and contents of the display annotations (as determined by the number of values in the second plurality) may be adjusted by a user or in response to a user's actions. This may allow a user to determine their preferred balance between the display annotation complexity, versus computational workload or responsiveness. A user may also deliberately impose constraints on the complexity of annotations to reduce cognitive load, ensuring that only the key features of the initial annotation data are represented in the display annotation data. For example, the resource constraint parameters may comprise parameters representing: an interaction mode for the display subregion; a field of view for the display subregion; a zoom level for the display subregion; a detail level; or a selected set of annotation classes.

For example, by changing the interaction mode, a user may change from a viewing mode to an editing mode. In the editing mode, it may be important to show a more complex set of annotations within the display subregion (requiring a higher number of values for the second plurality), while in the viewing mode, it may be more important to provide faster responsiveness at the cost of displaying a less complex set of annotations (i.e. constraining the second plurality of values to a lower number of values). The user may also be able to adjust the complexity of the display annotations directly by specifying a detail level. In some examples, the user may be able to modify the resource constraint parameters by adjusting the complexity and/or contents of the display annotations by selecting a set of annotation classes. For example, a user may wish to see only annotations classified as tumour cells, while hiding all other annotation classes or viewing annotations delineating cells as dots indicating location rather than polygons capturing cell morphology.

In step 106, instructions are received to select a subregion of the image as a display subregion. In use, such instructions may be generated by the display device in response to a user's inputs. For example, an initial display subregion may be displayed, and a user may provide instructions to zoom or pan to another region of the image.

In step 108, display annotation data is generated to represent the initial annotation data. The display annotation data corresponds to the display subregion and is defined by a second plurality of values. The second plurality of values is generated from the first plurality of values, and the number of values in the second plurality of values is determined with respect to the resource constraint parameters. For example, the second plurality of values may be selected as a subset of the first plurality of values, wherein the number of values in the second plurality is specified to be lower than a maximum value defined by e.g. the available accessible RAM of the display device.

In step 110, instructions are provided to display the display subregion overlaid with the display annotation data on the display device. In some examples, the instructions may be provided to an application or virtual machine running on the display device, such as a web browser. The instructions may then be used to display an image on a screen of the display device.

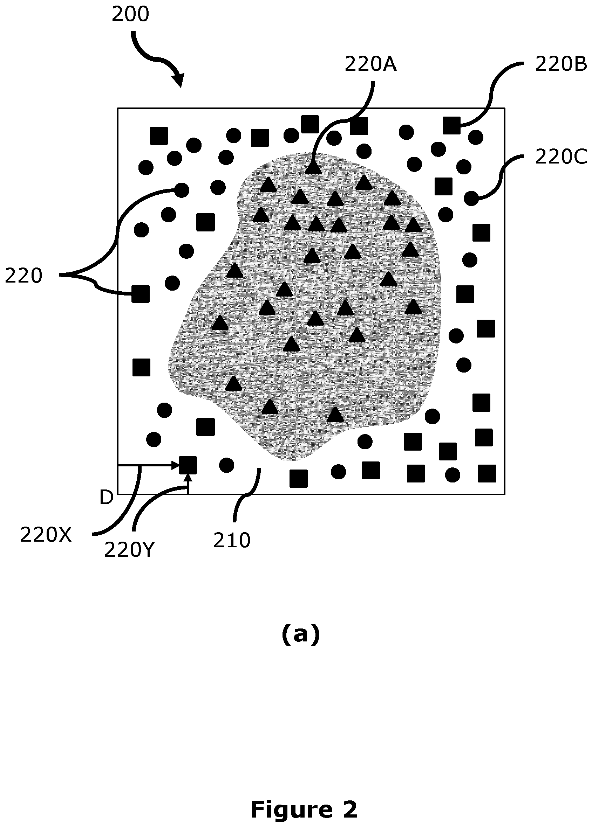

Figure 2A shows an example of a medical image data 200 comprising an image 210 and associated annotation data in the form of annotations 220. The complexity of the image 210, and the complexity and number of annotations 220 have been simplified for illustrative purposes. Real medical image data may comprise gigabytes' worth of high-resolution WSI images, and tens of thousands of accompanying annotations. The annotation data is defined by a first plurality of values. The first plurality of values includes, for each annotation: X-coordinate 220X and Y-coordinate 220Y values defining the position of the annotation on image 210 relative to a datum D; and a class value defining the class of the annotation. In the example shown, the annotation classes are represented by different shapes 220A, 220B, 220C.

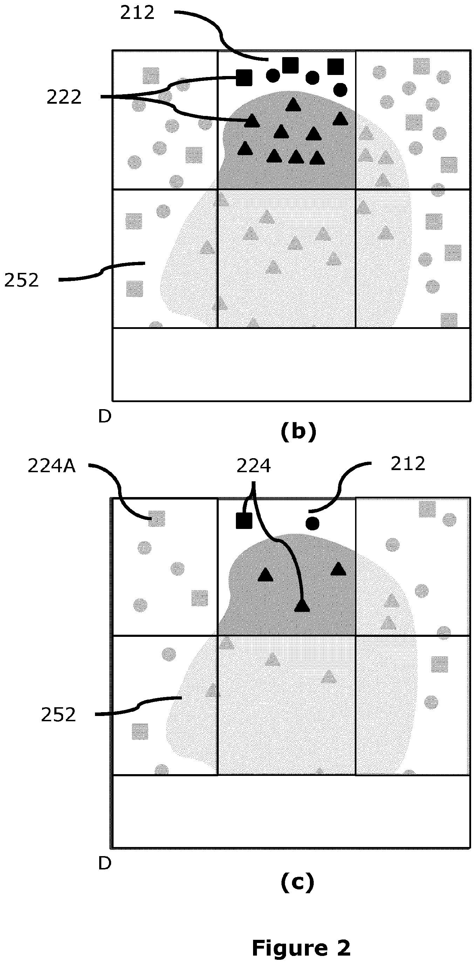

In Figure 2b, a display subsection 212 of the image 210 has been selected. The first step of generating the second plurality of values is then performed, by selecting an intermediate annotation data 222. The intermediate set of annotations 222 comprises all annotations 220 with an X-coordinate 220X and a Y-coordinate 220Y within the display subregion 212.

In addition to selecting the display subregion 212, in this example, the adjacent regions 252 of the image 210 are also identified. The adjacent regions 252 may be determined by identifying regions of the image 210 which border the display subregion 212. The adjacent regions 252 may be the same size as the display subregion 212, or they may be larger or smaller than the display subregion 252. The adjacent regions size may be determined by the resource constraint parameters, and/or the properties of the display device as well as the system interface design and parameters.

The values defining the intermediate set of annotations 222 are then used to generate the display annotation data 224, as shown in Figure 2c. The annotations in the display annotation data 224 are collectively representative of the initial annotation data 220 corresponding to the display subregion 212. In the example shown, the second plurality of values is generated by selecting the values defining a subset of the annotations from each of the different annotation classes 220A, 220B, 220C. Thus, the display annotation data 224 provides a similar spatial distribution of annotation classes 220A, 220B, 220C across the display subregion 212 as was provided by the initial annotation data 220. However, the number of annotations in the display annotation data 224 is smaller than the number of annotations in the initial annotation data 220, and hence the number of values in the second plurality of values is lower than the number of values in the first plurality of values. The number of values in the second plurality of values, and hence the number and complexity of annotations in the display annotation data 224, is determined at least by the resource constraint parameters.

If the adjacent regions 252 are also selected, the display annotation data can additionally comprise annotations 224A corresponding to the adjacent regions 252. That is, values are selected for the second plurality corresponding to annotations in the adjacent regions 252 as well as in the display subregion. As a result, the adjacent regions 252 and their display annotation data are already loaded and pre-processed, in case a user wishes to switch from the display subregion 212 to an adjacent region 252 by zooming, panning etc.

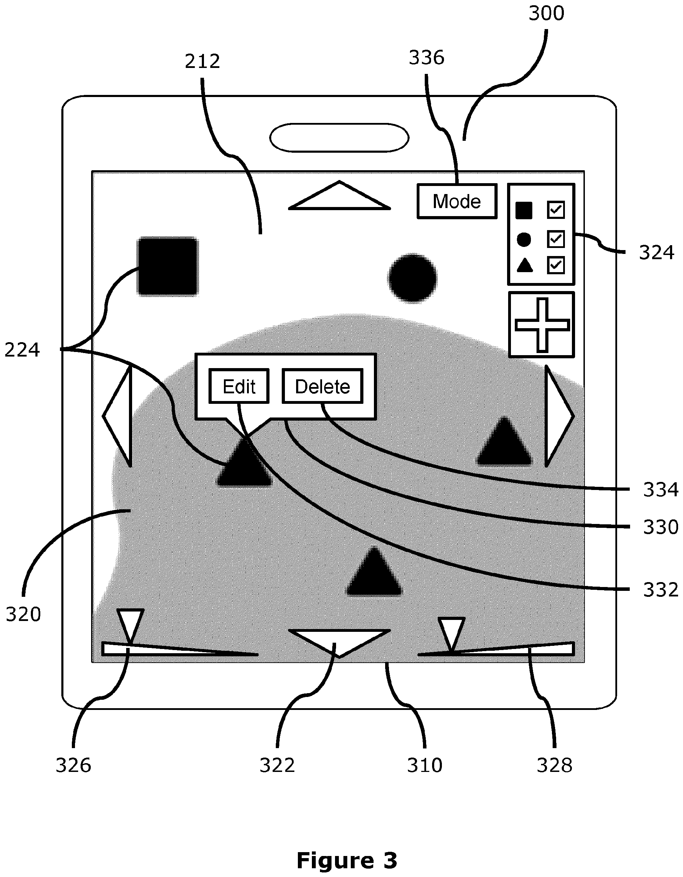

As shown in Figure 3, instructions are provided for displaying the display subregion 212 and the display annotation data 224 on a display device 300. The display device 300 comprises a touchscreen display element 310 allowing the display device 300 to additionally function as a user interface device. The user may use their finger or fingers to provide different kinds of input on the touchscreen element 310, as familiar to users of touchscreen devices.

A user interface 320 is provided to allow the user to provide inputs to the display device 300. The user interface 310 comprises selectable arrows 322, allowing a user to pan across the image 210 and view a new display subregion 212. Alternatively, a user may pan by swiping a finger across the touchscreen element 310, as familiar to users of touchscreen devices. 324 allows a user to selectively show and hide display annotation data 224 for different classes. Similarly, a zoom/FOV selector 326 allows a user to zoom in or out on image 210 or change the field of view of image 210. Alternatively, the user may zoom in or out using a two-finger pinch gesture on the touchscreen element 310, as familiar to users of touchscreen devices. A detail selector 328 allows a user to modify the complexity of the display annotation data 224. If the level of detail is increased, the number of values in the second plurality may increase and the number of annotations and/or complexity of the annotations shown in the display annotation data 224 may be increased. The maximum number of values in the second plurality may be capped according to the resource constraint parameters.

The user interface 320 also allows a user to provide modification instructions. A user can add a new annotation marker by tapping on the annotation addition button 325 and then tapping or drawing a region of the display subregion 212. A new annotation will then be created with an associated X-coordinate 220X and Y-coordinate 220Y value and optionally defined by geometry values defining a polygon corresponding to the drawing. A user can also select an existing annotation in the display annotation data 224 to edit it. An editing interface 330 will then appear 320 or the user can select an editing tool, with an edit button 332 allowing a user to edit properties of the selected annotation and a delete button 334 for deleting the selected annotation.

The display device 300 and user interface 320 may be configured to receive input via multi-touch and/or stylus with pressure and tilt input and haptic feedback. The display device 300 may be configured to differentiate different styluses and determine the appropriate action to perform. Actions can be drawing annotations (smooth stylus movement), editing an annotation (press and hold on an annotation), deleting an annotation (using the stylus as an eraser), deleting part of an annotation (erasing part with the stylus), reassigning an annotation to another type or class (hold and press), select multiple annotations (drawing with selection mode activated). Other inputs will be familiar to those skilled in the art.

The user interface 320 further comprises a mode switch interface 336. The mode switch interface 336 allows a user to alternate between different modes. For example, a user may switch from an editing mode, in which annotations can be viewed and their properties edited, to a viewing mode, in which annotations are visible but cannot be edited.

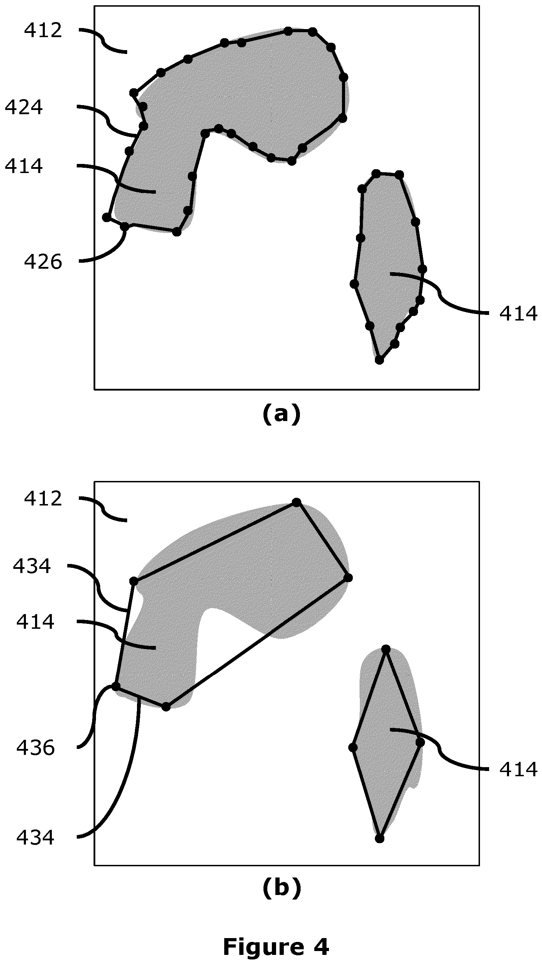

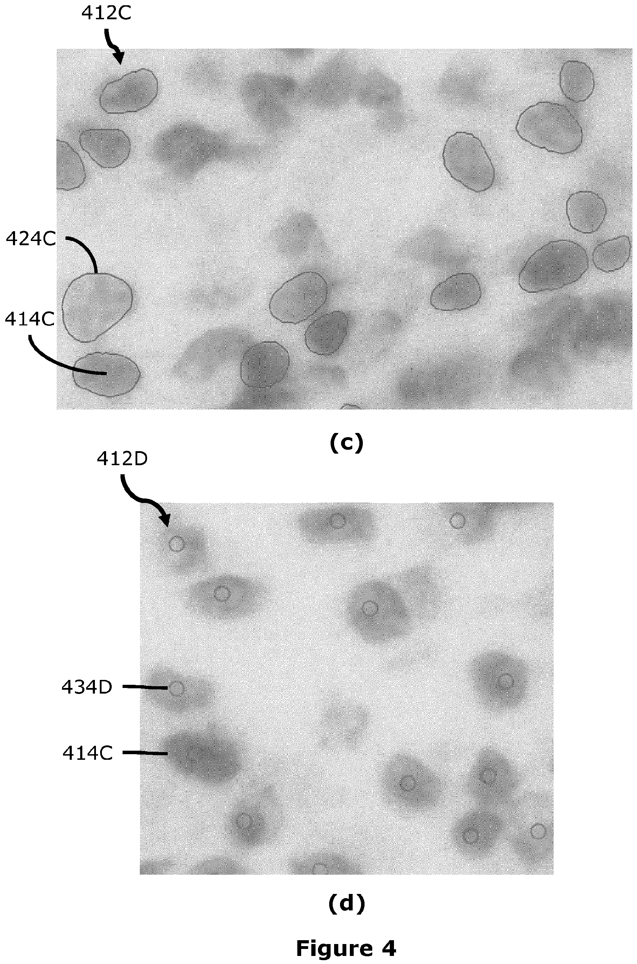

Figure 4 shows how an exemplary method may reduce the complexity of annotation data by simplifying the geometry of annotations. Figure 4a shows a display subregion 412, the display subregion including two tumour regions 414. The associated annotation data for the display subregion 412 comprises geometry values, the geometry values defining the location of vertices 426. The vertices 426 have been positioned so as to define the perimeters of two geometric annotations 424 which follow the geometry of the tumour regions 414. The geometric annotations 424 have a complex geometry due to the number of vertices 426.

To generate the second plurality of values, a new set of display vertices 436 is generated from the initial vertices 436. By reducing the number of vertices 436, simplified display geometric annotations 434 can be generated. The simplified display geometric annotations 434 still follow the approximate size and location of the tumour regions 414, but are defined by a smaller number of geometry values. The display vertices 436 may be generated by selecting a subset of the initial vertices 426, or new vertices 436 may be generated, e.g. by generating a new vertex 436 representing the average position of every n initial vertices 426.

Figure 4c and 4d illustrate another example of this technique using real medical image data. Figure 4c shows a display subregion of a medical image 412C comprising features 414C. In this case, the features 414C are individual cells. Initial annotation data is provided and shown in the form of geometric annotations 424C, with each geometric annotation being defined by vertices 426C and delimiting the morphology of a cell 414C.

Figure 4D shows how the complexity of the initial annotation data can be simplified by reducing the geometrical complexity of the annotations. Rather than displaying the geometric annotations 424C, a simplified annotation 424D in the form of a simple dot is generated to correspond with each annotated feature 414D. The position for the simplified annotation 424D can be calculated e.g. by calculating the average position of all the vertices 426C making up each geometric annotation 424C, such that the position of each simplified annotation 424D lies at the centroid of the corresponding geometric annotation 424C and marks the centre of the corresponding cell 414C.

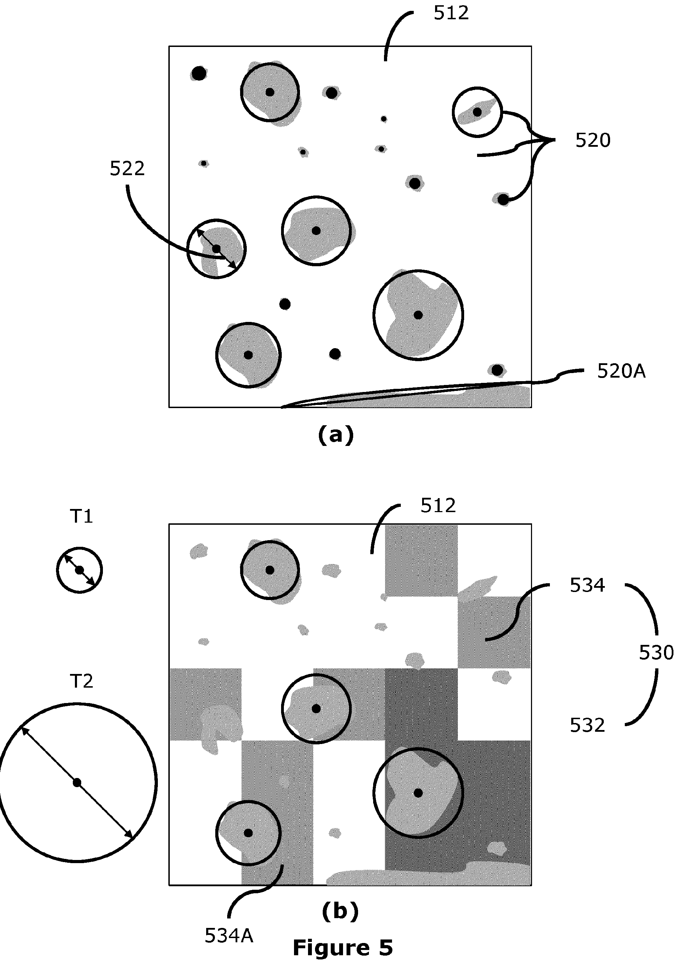

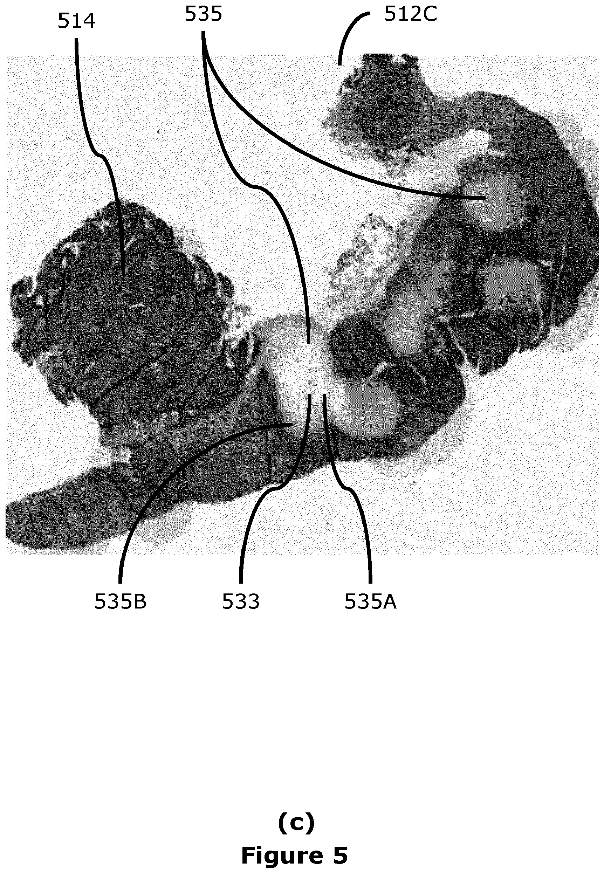

The number of values in the second plurality can also be reduced with reference to the size of the annotations, as shown in Figure 5.

Figure 5a shows an exemplary display subregion 512 and corresponding annotation data 520. The annotations 520 each have an associated overlay size value which defines their overlay size 522.

Figure 5b shows the display image 512 but overlaid with the display annotation data 530. The display annotation 530 data comprises both display annotations 532, and a spatial representation 534.

To generate the second plurality, the overlay size value 522 for each element is compared to a threshold range T1-T2. The values of the first plurality corresponding to annotations 520 in the initial annotation data with an overlay size value 522 within the threshold, i.e. with an overlay size 522 greater than the lower threshold T1 and smaller than the upper threshold T2 are selected for the second plurality. These values define the display annotations 530. Annotations 520 with a very large overlay size, such as the annotation 520A, are in this case deemed too large to usefully display as part of the display annotation data 530.

However, annotations 520 with an overlay size 522 smaller than the lower threshold T1 may be represented in the second plurality by a spatial representation 534 - in this case, a colourmap. The colourmap 534 comprises an array of elements 534A, wherein a colour value representing the shade of each element 534A is determined by the number of annotations 520 within the area of the display subregion 512 covered by the element 534A and having an overlay size 522 smaller than the lower threshold T1. Darker shades represent a higher number of annotations 520 within the area of the display subregion 512. The colourmap 534 thus allows a user to see the spatial density of small-size annotations 520, without having to zoom in further on the image and without the display device 300 having to individually render every annotation.

The threshold range T1-T2 is determined with reference to the resource constraint parameters. For example, if there is a high amount of available computational resources, the lower threshold T1 may be decreased so that more, smaller annotations are directly shown in the display annotation data 530.

Figure 5c shows another example of spatial representation. A display subregion 512C is shown comprising an imaged feature 514. The associated annotation data comprises a number of small, spatially concentrated annotations 533. To aid the user in locating and analysing the annotations 533, a spatial representation 535 in the form of a heat map 535 is generated and overlaid over the display subregion 512C. The colour of the heat map 535 is calculated by reference to the spatial density of the annotations 533. The heat map 535 has a lighter colour 535A in regions of high spatial density, and a darker colour 535B in regions of lower spatial density. Where there are no or minimal annotations within a given area, the colourmap 535 is transparent. The colours of the heat map 535 may be selected with reference to the dominant colours of the display subregion 513 or imaged feature 514, e.g. such that the heat map 535 contrasts with and is easily visible against the imaged feature 514.

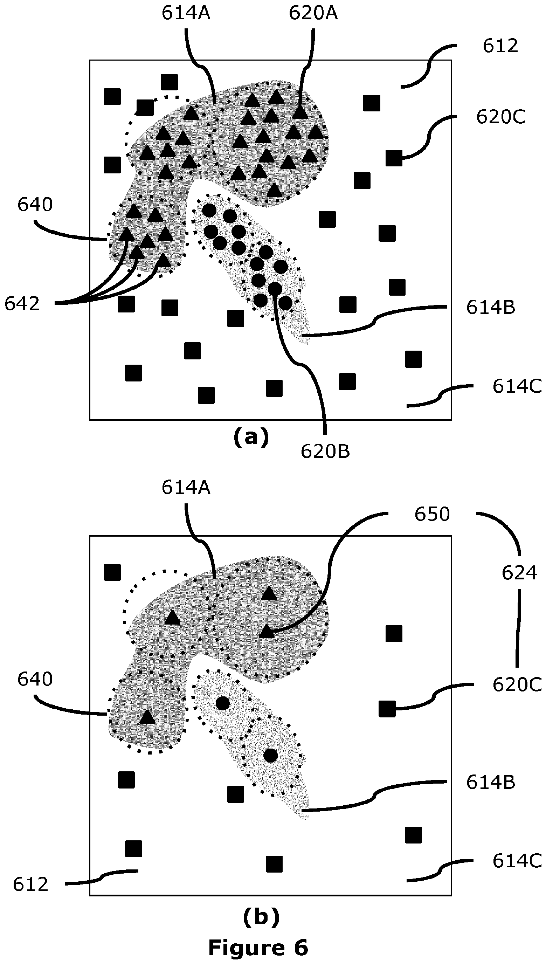

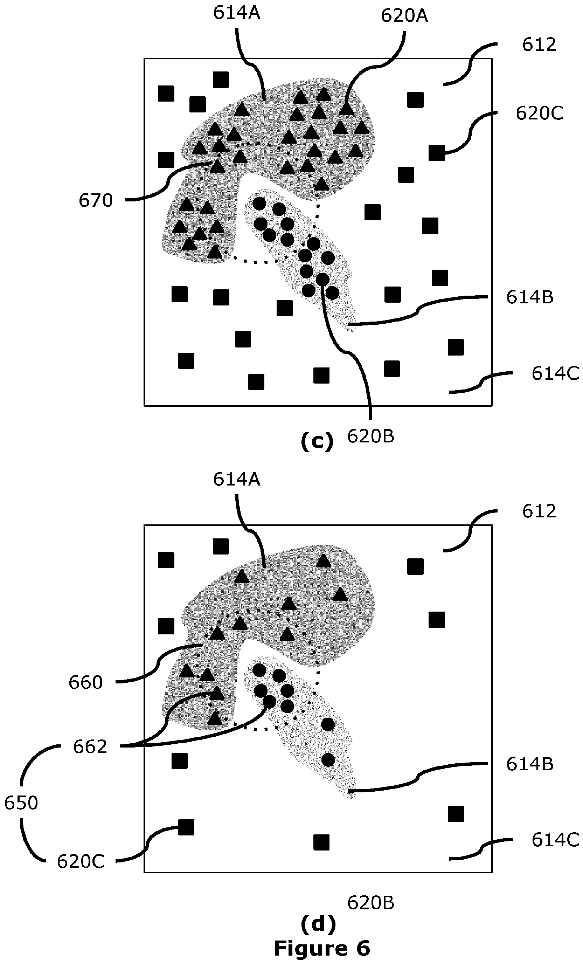

The number of values in the second plurality can also be reduced by identifying clusters of annotations, as shown in Figure 6. Figure 6a shows a display subregion 612. The display subregion comprises three different tissue regions 614A, 614B, 614C. The corresponding initial annotation data 620 for each region primarily comprises a different annotation class 620A, 620B, 620C representing the different dominant cell type within each of the tissue regions 614A, 614B, 614C.

The second plurality is generated by identifying clusters 640 of annotations 620 within the initial annotation data. Clusters 640 may be identified as areas where there is a higher than average spatial density of annotations 620, either annotations 620 of a particular class (in this case), or annotations 620 in general. A first cluster plurality of values is generated based on the contents of cluster 640. For example, the first cluster plurality of values may include the X-coordinate values, Y-coordinate values, and class values corresponding to all of the annotations 642 within cluster 640.

As shown in Figure 6b, once cluster 640 is identified and the first cluster plurality is generated, a second cluster plurality is generated from the first cluster plurality. The second cluster plurality may then be selected for the second plurality. The second cluster plurality may include values such as the average of all the X-coordinate values and Y-coordinate values of annotations 642 within cluster 640, and a value representing the median class value of annotations 642 within cluster 640. These values may be used to generate a cluster annotation 650 representing cluster 640 in the display annotation data 624. The boundary of cluster 640 as identified may also be shown as part of the annotation display data 650, to inform the user as to the size and geometry of cluster 640.

Alternatively or additionally, the second plurality may be generated by identifying intersection regions 660 in the initial annotation data 620, as shown in Figure 6c. An intersection region is where a first region primarily comprising a first class (or first composition of classes) intersects, boundaries or otherwise meets with a second region primarily comprising a second class (or second composition of classes). Intersection regions may be identified using the class values associated with the initial annotation data 620. For example in Figure 6c, it can be seen that there is an intersection region 660 where the first tissue region 614A comprising a first class of annotations 620A meets the second tissue region 614B comprising a second class of annotations 620B.

As shown in Figure 6d, the second plurality can be generated by selecting values representing a subset of the annotations 620 in the intersection region 660 having the first or second specified annotation class. In the case of Figure 6d, this entails selecting values within the intersection region 660 which correspond to annotations of the first 620A or second 620B class. The display annotation data 650 may thus specifically comprise annotations 662 within the intersection region 660. This ensures that the intersection between the first 614A and second 614B tissue regions can be clearly identified for subsequent analysis, even when the number and complexity of annotations in the display annotation data is decreased.

It will be understood that generating the second plurality may involve a combination of any of the previously described techniques. For example, the overlay size value may be used to filter annotations by size as previously described, while at the same time the class value is used to identify clusters or class regions, also as previously described.

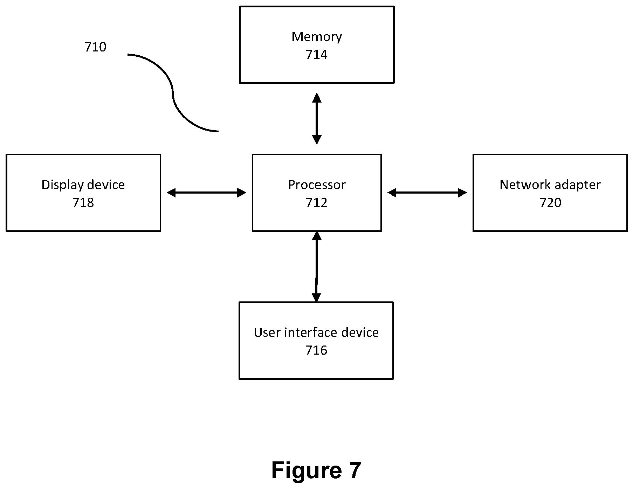

Figure 7 illustrates a schematic block diagram of a processing device for implementing the method. The processing device 710 comprises one or more processors 712 in communication with memory 714. The memory 714 is an example of a non-transitory computer readable storage medium. The one or more processors 712 are also in communication with one or more user interface devices 716 and one or more display elements 718. The various components of the processing device 710 may be implemented using generic means for computing known in the art. For example, the user interface devices 716 may comprise a keyboard or mouse and the display elements 718 may comprise a monitor, screen, or printer. The display elements 718 may be used to display image data and overlaid annotation data. The user may then provide modification and/or navigation instructions or another input into the memory 714 using the user interface device 716, for example by using a user interface to select a section of the display subregion to add an annotation, or select an annotation in order to modify a property of the annotation such as its overlay size or class.

In addition, the processing device 710 may comprise network access circuitry, such as a modem or network adaptor 720, to provide access to a network to obtain the medical image data. Alternatively, a different data entry and/or recording means, such as a portable disc drive or data interface may be provided in addition to, or instead of, the network adaptor 720 to provide the image data to the processing device 710 and/or record any modifications made by the user.

It will be appreciated that, in some examples, the various hardware units may be integrated with one another. For example, where the processing device 710 is provided by a tablet computer or cellular telephone, a display of the device 710 may provide both the display of the display device 718 and a touch sensor providing the user interface device 716.

In other examples, the various hardware units may be distributed across different physical devices. For example, the display device 718 and user interface device 716 may be integrated into a touchscreen-enabled display device, which receives display instructions from a remote processing unit comprising a processor 712, memory 712 and a network adapter 720. The methods described provide further advantages for such systems. Existing methods would require the processor 712 to generate display instructions for displaying the entire image and initial annotation data. These instructions would then be communicated via a wireless or wired communication to the display device. By contrast, the methods described reduce the amount of information which must be communicated from the processor 712 to the display device, reducing the burden on communication systems and improving latency.

## Claims

1. A method for processing medical image data for a display device, the method comprising:
receiving medical image data, the medical image data comprising at least one image and initial annotation data corresponding to the at least one image, the initial annotation data being defined by a first plurality of values;
receiving a set of resource constraint parameters;
receiving instructions to select a subregion of the image as a display subregion;
generating display annotation data to represent the initial annotation data, the display annotation data corresponding to the display subregion and being defined by a second plurality of values, wherein the second plurality is generated from the first plurality and the number of values in the second plurality is determined by the resource constraint parameters; and
providing instructions to display the display subregion overlaid with the display annotation data on the display device.

2. The method of claim 1, wherein the second plurality comprises a subset of the first plurality.

3. The method of any preceding claim, wherein the first plurality comprises overlay size values defining overlay sizes for annotations in the initial annotation data, and generating the second plurality comprises comparing the overlay size values to a threshold, the threshold being determined with reference to the resource constraint parameters.

4. The method of claim 3, wherein generating the second plurality comprises selecting, for the second plurality, values from the first plurality representing annotations with an overlay size value within the threshold.

5. The method of claim 3 or 4, wherein generating the second plurality comprises generating values defining a spatial representation, the spatial representation representing values from the first plurality representing annotations with an overlay size value lower than the threshold.

6. The method of any preceding claim, wherein the first plurality of values comprise geometry values defining geometries for annotations in the initial annotation data, and generating the lower-complexity annotation data comprises generating a lower-resolution set of geometry values for the annotations.

7. The method of any preceding claim, further comprising:
identifying a first cluster plurality of values from the first plurality representing a cluster of annotations within the annotation data, and
generating a second cluster plurality of values representing the cluster from the first cluster plurality.

8. The method of any preceding claim, wherein the first plurality comprises class values representing annotation classes in the initial annotation data, and the second plurality is generated with reference to the class values.

9. The method of claim 8, further comprising:
identifying, from the class values, a subset of classes of annotations within the initial annotation data; and
selecting for the second plurality, using the class values, values from the first plurality representing annotations within the subset of classes.

10. The method of claim 8 or 9, further comprising:
identifying, using the class values, a first class region within the display subregion comprising annotations having a first specified annotation class;
selecting for the second plurality, using the class values, values from the first plurality representing a subset of the annotations in the first class region having the first specified annotation class,
and preferably further comprising:
identifying, using the class values, an intersection subregion where the first class region intersects with a second class region, the second class region comprising annotations having a second specified annotation class,
wherein selecting values for the second plurality comprises selecting values representing a subset of the annotations in the intersection region having the first specified or second annotation class.

11. The method of any preceding claim, further comprising determining an adjacent subregion of the image to the selected display subregion, wherein the display annotation data further corresponds to the display subregion and the adjacent subregion.

12. The method of any preceding claim, further comprising:
receiving modification instructions from a user interface device;
associating the modification instructions with a value of the second plurality;
using the associated value of the second plurality, associating the modification with a value of the first plurality; and
modifying the associated value of the first plurality in response to the modification instructions.

13. A data processing device configured to carry out the method of any preceding claim.

14. A computer-readable storage medium comprising instructions which, when executed by a data processing device, cause the data processing device to carry out the method of any of claims 1-13.

15. A computer program comprising instructions which, when executed by a data processing device, cause the data processing device to carry out the method of any of claims 1 to 13.
